# EUROPÄISCHE PATENTSCHRIFT

(11) **EP 0 512 087 B1**
(45) Veröffentlichungstag und Bekanntmachung des Hinweises auf die Patenterteilung: **05.03.1997**
(21) Anmeldenummer: 91919945.5
(22) Anmeldetag: 21.11.1991
(51) Int. Cl.: C07K 1/00, C07K 2/00, C12N 9/72

(54) **VERFAHREN ZUR REAKTIVIERUNG VON DENATURIERTEM PROTEIN**
PROCESS FOR REACTIVATING DENATURED PROTEIN
PROCEDE POUR LA REACTIVATION DE PROTEINE DENATUREE

(30) Priorität: 22.11.1990 DE 4037196
(43) Veröffentlichungstag der Anmeldung: 11.11.1992
(73) Patentinhaber: BOEHRINGER MANNHEIM GMBH, 68298 Mannheim (DE)
(72) Erfinder: AMBROSIUS, Dorothea, D-8127 Iffeldorf (DE); RUDOLPH, Rainer, D-8120 Weilheim (DE)
(74) Vertreter: Huber, Bernhard, Dipl.-Chem.
(86) Internationale Anmeldenummer: EP9102190
(87) Internationale Veröffentlichungsnummer: WO9209622

(56) Entgegenhaltungen:
- EP-A- 219 874
- EP-A- 241 022
- EP-A- 364 926
- EP-A- 382 174

## Beschreibung

Die vorliegende Erfindung betrifft ein verbessertes Verfahren zur Solubilisierung und Renaturierung von denaturiertem Protein, wobei man das Protein mit einem Tris-Puffer behandelt, der eine Konzentration von mindestens 400 mmol/l an Tris-Base oder einem Salz von Tris aufweist.

Bei der Herstellung von Proteinen in prokaryontischen Zellen, wie z.B. E.coli, entstehen häufig schwer lösliche Protein-Aggregate (Inclusion Bodies). Um diese Proteine in ihre aktive Form zu überführen, sind Solubilisierungs- und Renaturierungsschritte erforderlich. Die Solubilisierung von Proteinen ist ein bekanntes Verfahren (s. z.B. EP-A 0 361 475, EP-A 0 114 506, EP-A 0 093 619 und EP-A 0 253 823).

Weiterhin sind sowohl Puffer als auch Verfahren zur Renaturierung von denaturierten Proteinen bekannt (s. z.B. WO 87/02673, EP-A 0 364 926, EP 0 241 022).

Ein wesentlicher, die Ausbeute an renaturiertem Protein limitierender Faktor bei der Reaktivierung von Proteinen (mit oder ohne Disulfidverbrückung) ist die Konkurrenzreaktion zwischen einer Überführung des denaturierten Proteins in das richtige Faltungsintermediat und einer Aggregation von mehreren Proteinmolekülen. Aus diesem Grund ist die Konzentration an denaturiertem Protein im Renaturierungspuffer ein wesentlicher Parameter für die Ausbeute des Renaturierungsverfahrens, d.h. mit steigender Konzentration an denaturiertem Protein wird die Aggregation begünstigt und die relative Ausbeute an renaturiertem Protein mit der Konformation des nativen Proteins sinkt.

Bei allen zur Zeit bekannten Verfahren zur Reaktivierung von Proteinen ist es daher notwendig, daß die Menge an denaturiertem Protein im Ansatz eine kritische Kozentration nicht überschreitet, Bei der oft geringen Löslichkeit des Proteins im verwendeten Reaktivierungspuffer ergeben sich daher erhebliche Nachteile bezüglich geringerer Ausbeute, höherem Zeitbedarf oder/und größerer Puffervolumina.

Eine Aufgabe der vorliegenden Erfindung ist es daher, Bedingungen für die Reaktivierung (d.h. insbesondere für die Solubilisierung und Renaturierung) zu schaffen, einen Puffer bereitzustellen, durch welche die Löslichkeit des denaturierten und renaturierten Proteins gegenüber bekannten Puffern erheblich gesteigert wird.

Diese Aufgabe wird erfindungsgemäß durch ein Verfahren zur Reaktivierung von denaturiertem Protein gelöst, welches dadurch gekennzeichnet ist, daß man das Protein mit einer Lösung von Tris (Hydroxymethyl) aminomethan-Base (im folgender als Tris bezeichnet) oder/und einem Salz von Tris mit einer Gesamtkonzentration an Tris von mindestens 400 mmol/l und einem pH-Wert inkubiert, bei dem das zu behandelnde Protein seine native Konformation einnehmen kann.

Durch Verwendung eines Puffers, welcher Tris-Base oder Salze von Tris mit einer Konzentration von mehr als 400 mmol/l enthält, wird bei der Reaktivierung von denaturierten Proteinen die Löslichkeit der renatuierenden Proteine erheblich gesteigert. Dies führt zu einer deutlichen Zunahme der Ausbeute an aktivem Protein im Vergleich zu bekannten Standardverfahren. Obwohl bisher bekannte Reaktivierungspuffer oftmals Tris in einer Konzentration von 50 bis 100 mmol/l zum Abpuffern der Reaktionslösung enthalten, wurde die überraschende Löslichkeits-vermittelnde (und damit die Renaturierausbeute verbessernde) Eigenschaft von Tris bei einer Konzentration von mindestens 400 mmol/l bisher nicht erkannt.

Als Salz von Tris im Sinne der vorliegenden Erfindung ist ein Tris-Salz einer beliebigen organischen oder anorganischen Säure zu verstehen. Beispiele für Tris-Salze sind etwa Tris-Acetat, Tris-Benzoat, Tris-Borat, Tris-Carbonat, Tris-Citrat, Tris-HCl, Tris-Maleat, Tris-Nitrat, Tris-Oxalat, Tris-Phosphat, Tris-Succinat, Tris-Sulfat und dergleichen.

Das erfindungsgemäße Verfahren ist zur generellen Renaturierung von Proteinen geeignet, wobei die Ursache der Denaturierung (Salz, Hitze etc.) an sich nicht kritisch ist. Obwohl das erfindungsgemäße Verfahren vorzugsweise für die Renaturierung gentechnologisch hergestellter und in inaktiver Form als Inclusion Body Material anfallender Produkte geeignet ist, läßt sich das Verfahren jedoch grundsätzlich auch bei anderen denaturierten Proteinen anwenden. Insbesondere ist das erfindungsgemäße Verfahren bei den in WO 87/02673, EP-A 0 241 022 und EP-A 0 364 926 offenbarten Verfahren anwendbar. Dabei offenbart die WO 87/02673 ein Verfahren zur Aktivierung von nicht glykosyliertem tPA nach Expression in Prokaryonten durch Zellaufschluß, Solubilisierung unter denaturierenden und reduzierenden Bedingungen und Reaktivierung unter oxidierenden Bedingungen in Gegenwart von GSH/GSSG, wobei man in der Stufe der Reaktivierung bei einem pH-Wert von 9 bis 12, einer GSH-Konzentration von 0,1 bis 20 mmol/l, einer GSSG-Konzentration von 0,01 bis 3 mmol/l und mit einer nicht denaturierenden Konzentration eines Denaturierungsmittels arbeitet. Die EP-A 0 241 022 offenbart ein Verfahren zur Renaturierung von denaturierten Proteinen in Lösung in einem Renaturierungspuffer, wobei man eine Lösung des zu renaturierenden Proteins in der im gewählten Puffer kritischen Konzentration herstellt und nach Bildung des Faltungsintermediats weiteres zu renaturierendes Protein in der zur Erzielung der kritischen Konzentration erforderlichen Menge zusetzt. Die EP-A 0 364 926 betrifft ein Verfahren zur Aktivierung von gentechnologisch hergestellten, in Prokaryonten exprimierten, biologisch aktiven Proteinen nach Zellaufschluß durch Solubilisierung unter denaturierenden und reduzierenden Bedingungen und nachfolgenden Reaktivierung unter oxidierenden und renaturierenden Bedingungen, wobei man bei einer Proteinkonzentration von 1 - 1000 µg/ml arbeitet und zwischen der Solubilisierung und der Reaktivierung eine Dialyse gegen einen Puffer mit einem pH-Wert zwischen 1 und 4, enthaltend 4 bis 8 mol/l Guanidin-Hydrochlorid oder 6 bis 10 mol/l Harnstoff, durchführt.

Das erfindungsgemäße Verfahren kann in zwei Varianten durchgeführt werden. Eine Variante ist es, in einem Tris-Puffer der angegebenen Konzentration zu arbeiten, so daß Tris oder/und ein Salz von Tris auch zur pH-Einstellung verwendet wird. Die zweite Variante ist, mit einem schon früher für das entsprechende Verfahren beschriebenen Puffer zu arbeiten und zusätzlich Tris oder/und ein Salz von Tris zuzusetzen. Dies bedeutet, daß der pH-Wert der Inkubationslösung durch eine von Tris verschiedene Puffersubstanz eingestellt wird. In beiden Fällen ist es zweckmäßig dafür zu sorgen, daß durch das Zusetzen von Tris bzw. durch die Erhöhung der Tris-Konzentration keine pH-Wert-Veränderung hervorgerufen wird.

Die Inkubation erfolgt bei einem pH-Wert, bei dem das zu behandelnde Protein in seiner nativen Konformation vorliegen kann. Dies bedeutet, daß die Inkubation bei dem erfindungsgemäßen Verfahren nicht bei einem solchen pH-Wert stattfindet, welcher die Ausbildung einer nativen Proteinkonformation unmöglich macht. Unter nativen Proteinkonformationen sind wiederum solche Sekundär-, Tertiär- und ggf. Quartärstrukturen zu verstehen, bei denen das Protein eine biologische Aktivität besitzen kann.

Das erfindungsgemäße Verfahren beinhaltet die Inkubation eines denaturierten Proteins mit einer Tris-Lösung, die eine Konzentration von mindestens 400 mmol/l aufweist. Vorzugsweise beträgt die Tris-Konzentration 0,4 bis 2 mol/l, besonders bevorzugt etwa 1 mol/l Tris. Bei einigen Proteinen (z.B. bei Antikörper-Fragmenten) werden optimale Reaktivierungsausbeuten bereits bei Tris-Konzentrationen im Bereich von 0,5 mol/l erreicht.

Die Ausbeute eines Renaturierungsprozesses hängt, wie oben bereits beschrieben, von der Konzentration des Proteins in der Renaturierungslösung ab. Für das erfindungsgemäße Verfahren wählt man vorzugsweise eine Proteinkonzentration im Bereich bis 4000 µg/ml. Abhängig von der Art des Proteins und des Renaturierungsverfahrens können sich jedoch auch Proteinkonzentrationen oberhalb dieses Bereichs als geeignet erweisen.

Bei dem erfindungsgemäßen Verfahren wird das denaturierte Protein entweder kontinuierlich oder chargenweise (z.B. Pulsnaturierung) der Renaturierungslösung zugesetzt. In vielen Fällen (z.B. bei der Renaturierung von tPA und tPA-Derivaten) hat es sich auch als günstig erwiesen, der Inkubationslösung 0,2 bis 1 mol/l Arginin zuzusetzen.

Bevorzugte Beispiele für Proteine, die durch das erfindungsgemäße Verfahren renaturiert werden können, sind rekombinanter tPA oder tPA-Muteine (insbesondere ein nicht glykosylierliertes tPA-Mutein mit der Domänenzusammensetzung K2P), rekombinanter Granulozyten-Kolonie-stimulierender Faktor (G-CSF) oder Antikörper oder deren Fragmente. Das erfindungsgemäße Verfahren ist jedoch nicht auf diese Beispiele beschränkt, sondern läßt sich auf beliebige Proteine übertragen.

Die Vorteile des erfindungsgemäßen Reaktivierungsverfahrens umfassen insbesondere eine Steigerung der Endausbeute an aktivem Protein um 30 bis 300 % gegenüber einem Verfahren, bei dem man mit einem Puffer arbeitet, welcher eine geringere Tris-Konzentration aufweist. Weiterhin kann die Konzentration an denaturiertem Protein im Renaturierungspuffer ohne Einbußen der Endausbeute erhöht werden, d.h. daß das Renaturierungsverfahren deutlich schneller und effektiver wird.

Insbesondere bringt das erfindungsgemäße Verfahren einen Vorteil bei der Pulsrenaturierung in einem Renaturierungsreaktor. Hier steigt die Ausbeute an renaturiertem Protein, weiterhin kann die Proteinkonzentration pro Puls gesteigert werden, woraus sich eine Verkürzung der Reaktivierungsdauer entwickelt. Es kann auch bis zu einer höheren Proteinendkonzentration im Renaturierungsansatz gepulst werden, ohne daß eine verringerte Renaturierungsausbeute beobachtet wird. Somit ergibt sich eine deutliche Verringerung des Puffervolumens. Besonders günstig hat sich ein Pulsrenaturierungsverfahren erwiesen, wobei man die Renaturierung in einem Arginin enthaltenden Puffer mit einer Tris-Konzentration von etwa 1 mol/l durchführt und pro Puls die Konzentration des zu renaturierenden Proteins um etwa 200 µg/ml erhöht.

Die Erfindung soll weiterhin durch die folgenden Beispiele verdeutlicht werden.

### Abkürzungen:

- GSH :: Glutathion reduziert
- GSSG :: Glutathion oxidiert
- t-PA :: tissue plasminogenaktivator
- Cprot :: Proteinkonzentration
- Arg :: Arginin
- Gdn :: Guanidin
- Renat. :: Renaturierung
- CK :: Creatinkinase

### Beispiel 1

Renaturierungsausbeute in Abhängigkeit von der Inkubationszeit bis zur Zugabe des zweiten Pulses (+/- 1 mol/l Tris)

### Ausgangsmaterial:

Inclusions Bodies (IB's) des tPA-Muteins K2P wurden nach WO 90/09437 (Beispiel 1) hergestellt und anschließend nach EP-A 0 361 475 A1 (Beispiel 1) solubilisiert und ins gemischte Disulfid überführt. Bei den Beispielen 2 bis 5 wurde mit dem Ausgangsmaterial analog verfahren.
- Renaturierung:: 0,6 mol/l Arginin/HCl, pH 8,5
1 mmol/l EDTA
0,7 mmol/l Glutathionin reduziert (GSH)
+/- 1 mol/l Tris
C_{Prot.} = 140 µg/ml pro Puls
- Zugabe 2. Puls:: 1 bis 9 h (siehe Tabelle)
- Inkubation:: 12 h bei Raumtemperatur nach Zugabe des letzten Pulses.

**Tabelle 1**

| Zeit 2. Puls (h) | ohne Tris Aktivität (+ Fibrin) (U/ml) | 1 mol/l Tris Aktivität (+ Fibrin) (U/ml) |
|---|---|---|
| 0 | 2855 | 4736 |
| 1 | 4671 | 9256 |
| 2 | 4974 | 9732 |
| 3 | 5060 | 10359 |
| 4 | 5233 | 9343 |
| 5 | 5168 | 9645 |
| 6 | 6845 | 10185 |
| 7 | 6152 | 9252 |
| 8 | 6814 | 10726 |
| 9 | 7115 | 10335 |

Aktivitätsbestimmung des renaturierten tPA-Muteins K2P und Definition der Einheit U ist bei Lill (ZGIMAL 42 (1987), 478-486) beschrieben.

Aus Tabelle 1 wird ersichtlich, daß im Arginin-Puffer ohne Tris ab einer Verweilzeit von > 6 h bis zur Zugabe des zweiten Pulses die maximale Renaturierungsausbeute erreicht wird. Fügt man dem Ansatz 1 mol/l Tris hinzu, dann treten folgende Änderungen auf: Die Maximalausbeute bei der Renaturierung steigt um 30 - 40 %; bereits nach einer Verweilzeit von 1 bis 3 h kann ohne Verringerung der Renaturierungsausbeute erneut denaturiertes Protein zugegeben werden.

### Beispiel 2

Renaturierung: Abhängigkeit von der Tris-Konzentration und dem Arg/Guanidin-Verhältnis

### Ausgangsmaterial:

Inclusions Bodies (IB's) des tPA-Muteins K2P, hergestellt wie in Beispiel 1 beschrieben.
- Renaturierung:: Versuch A: Trisabhängigkeit
0,6 mol/l Arginin/HCl, pH 8,5
1 mmol/l EDTA
0,7 mmol/l GSH
- Tris:: 0, 50, 100, 500, 1000 und 2000 mmol/l
C_{Prot.} = 80 µg/ml
- Inkubation:: 24 h bei Raumtemperatur
Versuch B: Puffer-Abhängigkeit
1 mmol/l EDTA, pH 8,5
0,7 mmol/l GSH
C_{Prot.} = 80 µg/ml
- Puffer:: (1) 0,6 mol/l Arginin/HCl
(2) 0,4 mol/l Arginin/HCl plus 0,2 mol/l Guanidin (Gdn)/HCl
(3) 0,2 mol/l Arginin/HCl plus 0,5 mol/l Guanidin (Gdn)/HCl jeweils +/- 1 mol/l Tris
- Inkubation:: 24 h bei Raumtemperatur

**Tabelle 2A**

| Versuch A: Trisabhängigkeit | |
|---|---|
| Tris (mmol/l) | Aktivität (+ Fibrin) (U/ml) |
| 0 | 2173 |
| 50 | 2750 |
| 100 | 2651 |
| 500 | 3902 |
| 1000 | 5339 |
| 2000 | 5131 |

**Tabelle 2B**

| Versuch B: Variation des Puffers | | | |
|---|---|---|---|
| Puffer | | Zusätze | Aktivität (+ Fibrin) (U/ml) |
| (1) | 0,6 mol/l Arg | - | 2047 |
| | | 1 mol/l Tris | 3553 |
| (2) | 0,4 mol/l Arg/ | - | 1577 |
| | 0,2 mol/l Gdn | 1 mol/l Tris | 4350 |
| (3) | 0,2 mol/l Arg/ | - | 1402 |
| | 0,5 mol/l Gdn | 1 mol/l Tris | 3277 |

Versuch A: Mit Erhöhung der Tris-Konzentration im Renaturierungspuffer steigt die Ausbeute. Das Optimum der Renaturierung liegt bei einer Tris-Konzentration von ca. 1 mol/l.

Zwischen 100 mmol/l und 500 mmol/l Tris nimmt die Renaturierungsausbeute überraschend zu. Versuch B: Durch Zusatz von 1 mol/l Tris wurde die Ausbeute in allen Fällen drastisch (Faktor 2 bis 3) erhöht.

### Beispiel 3

Renaturierung in Abhängigkeit von der Proteinkonzentration (+/- 1 mol/l Tris)

### Ausgangsmaterial:

Inclusions Bodies (IB's) des tPA-Muteins K2P, hergestellt nach Beispiel 1.
- Renaturierung:: Puffer A:
0,6 mol/l Arg/HCl, pH 8,5
1 mmol/l EDTA
0,7 mmol/l GSH
- C_{Prot.} :: 112, 223 und 446 µg/ml
Puffer B:
0,6 mol/l Arg/HCl, pH 8,5
1 mol/l Tris
1 mmol/l EDTA
0,7 mmol/l GSH
- C_{Prot.} :: 112, 223, 446, 893, 2230 und 4460 µg/ml
- Inkubation:: 24 h bei Raumtemperatur

Das Ergebnis dieses Experiments ist in der folgenden Tabelle 3 dargestellt.

**Tabelle 3**

| Tris (mol/l) | C_{Prot.} (µg/ml) | Aktivität (+ Fibrin) (U/ml) | Aktivität/C_{Prot.} (U/mg) |
|---|---|---|---|
| 0 | 112 | 2875 | 25670 |
| | 223 | 4187 | 18780 |
| | 446 | 5956 | 13350 |
| | | | |
| 1 | 112 | 2900 | 25890 |
| | 223 | 5171 | 23190 |
| | 446 | 10163 | 22790 |
| | 893 | 13286 | 14880 |
| | 2230 | 17483 | 7840 |
| | 4460 | 24930 | 5590 |

Im Argininpuffer ohne Tris fällt die Renaturierungsrate mit steigender Proteinkonzentration. Wird dem Puffer 1 mol/l Tris zugefügt, so ist bis zu einer Proteinkonzentration von 400 µg/ml keine signifikante Abnahme der Naturierungsausbeute (Aktivität/C_{Prot.}) meßbar. Erst bei höheren Proteinkonzentrationen fällt die Ausbeute mit steigender Konzentration.

### Beispiel 4

Pulsnaturierung: +/- 1 mol/l Tris

### Ausgangsmaterial:

Inclusions Bodies (IB's) des tPA-Muteins K2P, hergestellt nach Beispiel 1.
- Renaturierung:: 0,6 mol/l Arg/HCl, pH 8,5
1 mmol/l EDTA
0,7 mmol/l GSH
Tris: +/- 1 mol/l
C_{Prot.} = 150 µg/ml pro Puls
Verweilzeit: 12 h
Endkonzentration: 1500 µg/ml
Vorlage mit gemischten Disulfiden bei 0°C,
Reaktion unter Stickstoff

Das Ergebnis dieses Experiments ist in der folgenden Tabelle 4 dargestellt.

**Tabelle 4**

| Tris (mol/l) | C_{Prot.} (µg/ml) | Aktivität (+ Fibrin) (U/ml) | Aktivität/C_{Prot.} (U/mg) |
|---|---|---|---|
| 0 | 150 | 3000 | 20000 |
| | 450 | -- | |
| | 750 | 11622 | 15496 |
| | 1050 | 14430 | 13743 |
| | 1350 | 17507 | 12968 |
| 1 | 150 | 4353 | 29020 |
| | 450 | 10978 | 24395 |
| | 750 | 17319 | 23092 |
| | 1050 | 22600 | 21524 |
| | 1350 | 27920 | 20681 |

Beim Puls ohne Tris erfolgte ab einer Proteinkonzentration von 700 µg/ml eine deutliche Eintrübung, wohingegen der Puls mit 1 mol/l Tris-Zusatz selbst bei einer Proteinkonzentration von 1,5 mg/ml völlig klar war. Durch den Zusatz von 1 mol/l Tris wurde die Endausbeute um ca. 30 % gesteigert.

### Beispiel 5

Pulsnaturierung: Annäherung an eine kontinuierliches Verfahren (+/- 1 mol/l Tris)

### Ausgangsmaterial:

Inclusions Bodies (IB's) des tPA-Muteins K2P, hergestellt nach Beispiel 1.
- Renaturierung:: 0,6 mol/l Arg/HCl, pH 8,5
0,7 mmol/l GSH
1 mmol/l EDTA
+/- 1 mol/l Tris
Puls: Verweilzeiten: 30 min.
C_{Prot.} -Steigerung pro Puls:
(A) 9,3 µg/ml;
(B) 31 µg/ml
Zupumpdauer: 2 min.
Endkonzentration: 3000 µg/ml
Vorlage mit gemischten Disulfiden bei 0°C,
Reaktion unter N₂

Das Ergebnis dieses Experiments ist aus Tabelle 5 ersichtlich.

**Tabelle 5 A:**

| 1 mol/l Tris, C_{Prot.} = 9,3 µg/ml pro 30 min. | | | |
|---|---|---|---|
| Zeit (h) | C_{Prot.} (µg/ml) | Aktivität (+ Fibrin) (U/ml) | Aktivität/C_{Prot.} (U/mg) |
| 16 | 245 | 10223 | 41726 |
| 23 | 527 | 23295 | 44203 |
| 42 | 800 | 30405 | 38006 |
| 48 | 904 | 43339 | 47941 |
| 69 | 1299 | 43964 | 33844 |
| 73 | 1375 | 49505 | 36003 |
| 137 | 2580 | 107499 | 41666 |
| 146 | 2759 | 104009 | 37698 |

| ohne Tris, C_{Prot.} = 9,3 µg/ml pro 30 min. | | | |
|---|---|---|---|
| Zeit (h) | C_{Prot.} (µg/ml) | Aktivität (+ Fibrin) (U/ml) | Aktivität/C_{Prot.} (U/mg) |
| 8 | 114 | 2932 | 25719 |
| 24 | 338 | 5635 | 16671 |
| 40 | 563 | 14723 | 26150 |
| 56 | 788 | 24035 | 30501 |
| 72 | 1013 | 27909 | 27550 |
| 88 | 1238 | 33802 | 27303 |
| 104 | 1463 | 37734 | 25792 |
| 120 | 1688 | 46420 | 27500 |
| 176 | 2485 | 59195 | 23821 |
| 192 | 2710 | 62098 | 22914 |
| 208 | 2935 | 61764 | 21044 |
| 224 | 3160 | 65896 | 20853 |

**Tabelle 5 B:**

| 1 mol/l Tris, C_{Prot.} = 31 µg/ml pro 30 min. | | | |
|---|---|---|---|
| Zeit (h) | C_{Prot.} (µg/ml) | Aktivität (+ Fibrin) (U/ml) | Aktivität/C_{Prot.} (U/mg) |
| 1 | 94 | 1590 | 16914 |
| 3 | 218 | 6265 | 28738 |
| 18 | 1094 | 33897 | 30984 |
| 42 | 2563 | - | - |
| 49 | 3031 | 53033 | 17496 |
| 65 | 3696 | 72056 | 19495 |

| ohne Tris, C_{Prot.} = 31 µg/ml pro 30 min. | | | |
|---|---|---|---|
| Zeit (h) | C_{Prot.} (µg/ml) | Aktivität (+ Fibrin) (U/ml) | Aktivität/C_{Prot.} (U/mg) |
| 1 | 94 | 107 | - |
| 3 | 219 | 4587 | 20945 |
| 18 | 1094 | 20257 | 18516 |
| 42 | 2563 | 23580 | 9200 |
| 49 | 3031 | 33482 | 11046 |
| 65 | 3696 | 36126 | 9774 |

Eine Annäherung an ein kontinuierliches Verfahren (Erhöhung der Zugabefrequenz der einzelnen Naturierungspulse bei sonst entsprechenden Bedingungen) ergibt gleiche Naturierungsraten im Vergleich zum Pulsexperiment. Durch Zusatz von 1 mol/l Tris zum Renaturierungspuffer wird generell die Endausbeute um ca. 20 % bis 100 % erhöht.

### Beispiel 6

Renaturierung von reduziertem K2P in Abhängigkeit von der Tris-Konzentration

Ausgangsmaterial: Inclusions Bodies (IB's) des tPA-Muteins K2P, hergestellt nach Beispiel 1.
- Solubilisierung:: 6 mol/l Gdn/HCl, pH 8,3
0,1 mol/l Tris
1 mmol/l EDTA
0,1 mol/l DTE
C_{Prot.} = 7,5 mg/ml
2 min. Ultraturrax
Inkubation: 1 h bei Raumtemperatur
Abstoppen: pH 3,0 mit HCl
- Dialyse:: 6 mol/l Gdn/HCl, pH 3,0
1 mmol/l EDTA
- Renaturierung:: 0,7 mol/l Arg/HCl, pH 8,5
1 mmol/l EDTA
3 mmol/l GSH
0,3 mmol/l GSSG (Glutathion oxidiert)
Tris: 0; 0,3; 0,6; 0,9; 1,2; 1,5; 1,8 und 2,1 mol/l
C_{Prot.} = 150 µg/ml

Das Ergebnis ist aus der Tabelle 6 ersichtlich.

**Tabelle 6:**

| Tris (mol/l) | Aktivität (U/ml) |
|---|---|
| 0 | 1339 |
| 0,3 | 1478 |
| 0,6 | 1591 |
| 0,9 | 1725 |
| 1,2 | 1766 |
| 1,5 | 1766 |
| 1,8 | 1725 |
| 2,1 | 1717 |

Auch bei der direkten Renaturierung des reduzierten Proteins wird durch Zusatz von ≥ 1 mol/l Tris die Renaturierungsausbeute um 30 % gesteigert.

### Beispiel 7

Renaturierung von G-CSF

### Ausgangsmaterial:

Inclusion Bodies von G-CSF, hergestellt nach PCT/EP91/00192 (Beispiel 3)
- Solubilisierung:: 6 mol/l Gdn/HCl, pH 8
0,1 mol/l Tris
1 mmol/l EDTA
0,1 mol/l DTE
C_{Prot.} = 10 mg/ml
2 min. Ultraturrax
Inkubation: 2 h bei Raumtemperatur unter Rühren
Abstoppen: pH 3 mit HCl
- Dialyse:: 6 mol/l Gdn/HCl, pH 2,5
3 mmol/l EDTA
4°C, bis Reduktionsmitttel völlig entfernt

Proteinkonzentration nach Dialyse: 8,5 mg/ml
- Renaturierung:: Versuch I:
0,6 mol/l Arg/HCl, pH 8
1 mmol/l EDTA
0,5 mmol/l GSH
5 mmol/l GSSG
A) 0,1 mol/l Tris
B) 1 mol/l Tris

Puls: C = 50 µg/ml in 30 min.
t = 1 h
Proteinendkonz. = 1 mg/ml
Versuch II: 0,6 mol/l Arg/HCl, pH 8
1 mmol/l EDTA
0,5 mmol/l GSH
5 mmol/l GSSG
A) 0,1 mol/l Tris
B) 1 mol/l Tris

Puls: C = 50 µg/ml in 30 min.
t = 1 h
Proteinkonz. = 0,6 mg/ml

Nach der Renaturierung erfolgt eine Zentrifugation bei 16000 rpm, 30 min. Daran anschließend erfolgt eine Dialyse gegen 20 mmol/l Tris, pH 8 1 mmol/l EDTA

Das Ergebnis ist aus Tabelle 7 ersichtlich.

**Tabelle 7**

| Versuch | % Renaturierung nach Dialyse in Tris-Puffer pH 8,0 |
|---|---|
| Versuch I | |
| A: 0,1 mol/l Tris | 10 |
| B: 1 mol/l Tris | 63 |
| | |

| Versuch II | |
|---|---|
| A: 0,1 mol/l Tris | 20 |
| B: 1 mol/l Tris | 50 |

Die Aktivitätsbestimmung von G-CSF erfolgte mit Hilfe eines Proliferationstests (³H-Thymidineinbau) mit einer G-CSF abhängigen murinen Leukämie Zellinie (NFS60) wie in Mossmann, T. (1985) J. Immunol.Methods 65, 66-73 und Holmes, K.L.; Plaszynski, E.; Frederickson, T.T.; Morse, H.C. III & Ihle, J. (1985) PNAS USA 82, 6687-6691 beschrieben. Analoge Ergebnisse werden mit nach EP 91 107 429.2 hergestelltem G-CSF und G-CSF-Muteinen erhalten.

### Beispiel 8

Renaturierung von Antikörper Fab-Fragmenten
- Denaturierung:: 5 mol/l Gdn/HCl, pH 8,5
0,1 mol/l Tris
2 mmol/l EDTA
0,3 mol/l DTE
C_{Prot.} = 5 mg/ml
Inkubation: 2 - 3 h bei Raumtemperatur
- Renaturierung:: Tris-Puffer, pH 7,5
(Tris: 0,05; 0,1; 0,2; 0,3; 0,5; 0,75
und 1 mol/l)
2 mmol/l EDTA
6 mmol/l GSSG
C_{Prot.} = 50 µg/ml
Inkubation: 80 h bei 10°C

Aktivitätstest: ELISA mit biotinylierter CK nach DE-A 38 35 350.4 (Beispiel 8.2).

Das Ergebnis ist aus Tabelle 8 ersichtlich.

**Tabelle 8**

| Tris (mol/l) | % Renaturierung |
|---|---|
| 0,05 | 13 |
| 0,1 | 18 |
| 0,2 | 22 |
| 0,3 | 27 |
| 0,5 | 35 |
| 0,75 | 36 |
| 1 | 37 |

Die Renaturierungsausbeute der Antikörper-Fab's steigt linear bis zu einer Tris-Konzentration von 0,5 mol/l; durch eine weitere Erhöhung der Tris-Konzentration wird die Ausbeute nur noch gering verbessert.

## Patentansprüche

1. Verfahren zur Reaktivierung von denaturiertem Protein,
**dadurch gekennzeichnet**,
daß man das Protein mit einer Lösung von Tris-Base oder/und einem Salz von Tris mit einer Gesamtkonzentration an Tris von mindestens 400 mmol/l und einem pH-Wert inkubiert, bei dem das zu behandelnde Protein seine native Konformation einnehmen kann.

2. Verfahren nach Anspruch 1,
**dadurch gekennzeichnet**,
daß der pH-Wert der Inkubationslösung durch eine von Tris verschiedene Puffersubstanz eingestellt wird.

3. Verfahren nach Anspruch 1,
**dadurch gekennzeichnet**,
daß Tris auch zur pH-Wert Einstellung verwendet wird.

4. Verfahren nach einem der vorhergehenden Ansprüche,
**dadurch gekennzeichnet**,
daß man 0,4 bis 2 mol/l Tris-Lösung verwendet.

5. Verfahren nach einem der vorhergehenden Ansprüche,
**dadurch gekennzeichnet**,
daß man der Inkubationslösung 0,2 bis 1,0 mol/l Arginin zusetzt.

6. Verfahren nach Anspruch 5,
**dadurch gekennzeichnet**,
daß man rekombinantes tPA oder ein tPA-Mutein behandelt.

7. Verfahren nach Anspruch 6,
**dadurch gekennzeichnet**,
daß man nicht glykosyliertes tPA-Mutein der Domänenzusammensetzung K2P behandelt.

8. Verfahren nach einem der vorhergehenden Ansprüche,
**dadurch gekennzeichnet**,
daß das denaturierte Protein kontinuierlich oder chargenweise der Renaturierungslösung zugesetzt wird.

9. Verfahren nach Anspruch 8,
**dadurch gekennzeichnet**,
daß man ein Pulsrenaturierungsverfahren in einem Renaturierungsreaktor durchführt.

10. Verfahren nach Anspruch 9,
**dadurch gekennzeichnet**,
daß man die Renaturierung in einem Arginin enthaltenden Puffer mit einer Tris-Konzentration von etwa 1 mol/l durchführt und pro Puls die Proteinkonzentration um etwa 200 µg erhöht.

11. Verfahren nach einem der Ansprüche 1 bis 5 oder 8 bis 10,
**dadurch gekennzeichnet**,
daß man rekombinantes G-CSF behandelt.

12. Verfahren nach einem der Ansprüche 1 bis 5 oder 8 bis 10,
**dadurch gekennzeichnet**,
daß man Antikörper oder deren Fragmente behandelt.

## Claims

1. Process for the reactivation of denatured protein, **wherein** the protein is incubated with a solution of Tris base or/and a salt of Tris at a total concentration of Tris of at least 400 mmol/l and at a pH value at which the protein to be treated can take up its native conformation.

2. Process as claimed in claim 1, **wherein** the pH value of the incubation solution is adjusted by a buffer substance which is different from Tris.

3. Process as claimed in claim 1, **wherein** Tris is also used to adjust the pH value.

4. Process as claimed in one of the previous claims, **wherein** a 0.4 to 2 mol/l Tris solution is used.

5. Process as claimed in one of the previous claims, **wherein** 0.2 to 1.0 mol/l arginine is added to the incubation solution.

6. Process as claimed in claim 5, **wherein** recombinant tPA or a tPA mutein is treated.

7. Process as claimed in claim 6, **wherein** a non-glycosylated tPA mutein having the domain composition K2P is treated.

8. Process as claimed in one of the previous claims, **wherein** the denatured protein is added continuously or batchwise to the renaturation solution.

9. Process as claimed in claim 8, **wherein** a pulse renaturation process is carried out in a renaturation reactor.

10. Process as claimed in claim 9, **wherein** the renaturation is carried out in a buffer containing arginine at a Tris concentration of about 1 mol/l and the protein concentration is increased by about 200 µg per pulse.

11. Process as claimed in one of the claims 1 to 5 or 8 to 10, **wherein** recombinant G-CSF is treated.

12. Process as claimed in one of the claims 1 to 5 or 8 to 10, **wherein** antibodies or fragments thereof are treated.

## Revendications

1. Procédé de réactivation de protéine dénaturée, caractérisé en ce que l'on incube la protéine avec une solution de tris-base et/ou d'un sel de tris ayant une concentration totale en tris d'au moins 400 mmol/l et un pH auquel la protéine à traiter peut revêtir sa conformation native.

2. Procédé selon la revendication 1, caractérisé en ce que le pH de la solution d'incubation est réglé par une substance tampon différente de tris.

3. Procédé selon la revendication 1, caractérisé en ce que tris peut aussi être utilisé pour le réglage du pH.

4. Procédé selon l'une des revendications précédentes, caractérisé en ce que l'on utilise une solution de tris à 0,4 à 2 mol/l.

5. Procédé selon l'une des revendications précédentes, caractérisé en ce que l'on ajoute à la solution d'incubation 0,2 à 1,0 mol/l d'arginine.

6. Procédé selon la revendication 5, caractérisé en ce que l'on traite le tPA recombiné ou une mutéine du tPA.

7. Procédé selon la revendication 6, caractérisé en ce que l'on traite une mutéine du tPA non glycosylée de composition des domaines K2P.

8. Procédé selon l'une des revendications précédentes, caractérisé en ce que la protéine dénaturée est ajoutée de manière continue ou discontinue à la solution de renaturation.

9. Procédé selon la revendication 8, caractérisé en ce que l'on met en oeuvre un procédé de renaturation par impulsions dans un réacteur de renaturation.

10. Procédé selon la revendication 9, caractérisé en ce que l'on réalise la renaturation dans un tampon contenant de l'arginine avec une concentration en tris d'environ 1 mol/l et l'on augmente la concentration en protéine d'environ 200 µg par impulsion.

11. Procédé selon l'une des revendications 1 à 5 ou 8 à 10, caractérisé en ce que l'on traite du G-CSF recombiné.

12. Procédé selon l'une des revendications 1 à 5 ou 8 à 10, caractérisé en ce que l'on traite des anticorps ou leurs fragments.
